# EUROPÄISCHE PATENTSCHRIFT

(11) **EP 0 810 193 B1**
(45) Veröffentlichungstag und Bekanntmachung des Hinweises auf die Patenterteilung: **20.06.2001**
(21) Anmeldenummer: 97105725.2
(22) Anmeldetag: 07.04.1997
(51) Int. Cl.: C07C 29/70

(54) **Verfahren zur katalytischen Herstellung von Alkalialkoholaten**
Process for the catalytic preparation of alkali metal alcoholates
Procédé pour la préparation catalytique d'alcoolats de métaux alcalins

(30) Priorität: 29.05.1996 DE 19621466
(43) Veröffentlichungstag der Anmeldung: 03.12.1997
(73) Patentinhaber: Degussa AG, 40474 Düsseldorf (DE)
(72) Erfinder: Hamann, Carl Heinz, Prof. Dr., 26939 Ovelgönne (DE); Schmittinger, Peter, Dr., 53859 Niederkassel (DE)

(56) Entgegenhaltungen:
- EP-A- 0 177 768
- DE-C- 593 385
- US-A- 2 069 403
- US-A- 2 336 045
- US-A- 2 761 880
- US-A- 5 262 133

## Beschreibung

Die Erfindung betrifft ein Verfahren zur katalytischen Herstellung von Alkalialkoholaten aus Alkaliamalgamen und Alkoholen.

Alkalialkoholate, besonders solche, deren Alkoholkomponente bis zu 4 Kohlenstoffatome enthält, sind wertvolle Chemikalien. Sie werden z. B. als Katalysatoren bei der Synthese vieler organischer Verbindungen verwendet. Dabei haben vorzugsweise die Alkoholate des Natriums und des Kaliums praktische Bedeutung erlangt. Zur Darstellung von Alkalialkoholaten sind mehrere Methoden bekannt [F. A. Dickes, Ber. Dtsch. Chem. Ges. 63, 2753 (1930)]. So enthalten Lösungen von Alkalihydroxiden in einem Alkohol Alkalialkoholat im Gleichgewicht. Durch z. B. destillative Entfernung des in diesem Gleichgewicht befindlichen Wassers gelangt man zu reinen Alkalialkoholaten. Besonders bei niedrig siedenden Alkoholen wird für diese Art der Gleichgewichtsverschiebung jedoch sehr viel Energie benötigt.

Auf direktem Wege kommt man zu reinen Alkalialkoholaten, indem man ein Alkalimetall in dem entsprechenden Alkohol auflöst. Dabei reagieren Natrium und Kalium mit niederen aliphatischen Alkoholen, wie Methanol und Ethanol, stürmisch unter Wasserstoffentwicklung. Höhere Alkoholate, wie z. B. Propanole und Butanole, setzt man vorzugsweise oberhalb des Schmelzpunktes der Alkalimetalle, gegebenenfalls unter Druck und Rühren, mit diesen um. Die Methode der direkten Herstellung der Alkalialkoholate aus Metall und Alkohol kommt für ein kommerzielles Verfahren kaum in Frage, weil die hierfür als Ausgangsmaterialien benötigten Alkalimetalle zu teuer sind.

Wirtschaftlicher ist es, als Alkaliquelle das flüssige, bei der Chloralkali-Elektrolyse nach dem Quecksilber-Verfahren anfallende Alkaliamalgam einzusetzen.

Die Umsetzung von Alkaliamalgam mit Alkoholen sowie die Verwendung von Katalysatoren für diese Reaktion sind bekannt.

R. B. Mac Mullin, Chemical Engineering Progress, Sept. 1950, S. 440, erwähnt unter anderem die Umsetzung von Alkaliamalgam mit Methanol in einem Reaktor, welcher Graphit als Kontakt enthält. In den US-PSS 2 336 045 und 2 761 880 werden nicht amalgamierende Stoffe, wie z. B. Eisen, Graphit oder Mischungen davon, als Kontakt vorgeschlagen.

In der US-PS 2 069 403 werden als Katalysatoren auch Metallgitter, bestehend aus Schwermetallegierungen, beschrieben.

Mit dem Verfahren nach dem europäischen Patent 0 177 768 gelingt es, die Reaktion zwischen dem Amalgam und dem Alkohol wesentlich zu beschleunigen. Es wird für die Reaktion eine Schüttung aus stückigem Anthrazit verwendet, deren Oberfläche mit Schwermetalloxid oder einer Mischung aus Schwermetalloxiden belegt ist. Amalgam und Alkohol werden nach dem Gegenstromprinzip kontinuierlich eingespeist, die Produkte kontinuierlich abgezogen.

Die US-A-5 262 133 beschreibt ein zweistufiges Verfahren zur Herstellung von Alkoholaten unter Verwendung verschiedener Katalysatoren, u.a. Wolframcarbid.

Besonders die Kombination der Oxide von Nickel und Molybdän zeichnen sich dabei durch eine hohe Aktivität aus.

Es werden vorzugsweise aliphatische Alkohole mit 1 bis 4 Kohlenstoffatomen umgesetzt. Es können jedoch auch andere Alkohole bei diesem Verfahren als Ausgangsmaterial verwendet werden, wie z. B. aliphatische Alkohole mit mehr als 4 Kohlenstoffatomen.

Der damit beschriebene Stand der Technik ist allerdings nach wie vor unbefriedigend. Z. B. werden nur 60 bis 70 % des mit dem Amalgam eingetragenen Natriums mit Methanol umgesetzt. Eine zweite Verfahrensstufe zum Entfernen des restlichen Alkalimetalls aus dem Amalgam bleibt so unentbehrlich. Für den Fall des Umsatzes von Natriumamalgam mit Ethanol liegt die sogenannte Zehrung nur noch bei 40 bis 50 %. Des weiteren ist die Standzeit des oxidbeschichteten Kontaktes unbefriedigend. Die Zehrung sinkt im Verlauf von etwa einem Jahr soweit ab, daß der Kontakt erneuert werden muß.

Es bestand somit die Aufgabe, ein Verfahren zu entwickeln, welches die angeführten Nachteile vermeidet. Diese Aufgabe wurde gemäß der Patentansprüche gelöst.

Es wurde ein Verfahren zur katalytischen Herstellung von Alkalialkoholaten aus Alkaliamalgamen und Alkoholen gefunden, in dem die Umsetzung in Gegenwart von Katalysatoren aus Carbiden und Nitriden der Metalle Chrom und Molybdän, Wolframnitrid sowie Titancarbid durchgeführt wird.

Nach dem erfindungsgemäßen Verfahren werden vorzugsweise aliphatische Alkohole mit 1 bis 4 Kohlenstoffatomen umgesetzt. Es können jedoch auch andere Alkohole bei diesem Verfahren als Ausgangsmaterial verwendet werden, wie z. B. aliphatische Alkohole mit mehr als 4 Kohlenstoffatomen.

Die Reaktion zwischen dem Amalgam und dem Alkohol läuft kontinuierlich oder diskontinuierlich mit hoher Geschwindigkeit, so daß hohe Raum-Zeit-Ausbeuten erreicht werden.

Es wurde gefunden, daß die Zersetzung von Alkaliamalgam durch Carbide und Nitride von Metallen der Gruppe VIa des Periodensystems sowie Titancarbid noch einmal wesentlich besser beschleunigt wird, als durch die derzeit eingesetzten Schwermetalloxide. Bei Benutzung von stückigem Vollmaterial aus diesen Stoffen im Beispiel 1 charakterisierten Modellreaktor wurden für den Fall der Umsetzung von Natriumamalgam mit Methanol die folgenden Reaktionszeiten beobachtet: Wolframcarbid 0,6 min, Chromnitrid 1 min, Titancarbid 1,7 min, Molybdäncarbid 1,8 min, Chromcarbid 2,2 min.

Für einen praktischen Einsatz sind aus Kostengründen Trägermaterialien für den gewählten Katalysator erforderlich.

Benutzt man für den Fall von Carbiden ein Trägermaterial aus Kohlenstoff (Petrolkoks, Graphit, Anthrazit) und stellt die Beschichtung durch Reaktion von Molybdän mit dem Kohlenstoff her, so entsteht eine besonders innige Verbindung zwischen Träger und aktiver Schicht [A. J. Hegedüs, J. Neugebauer, Z. Anorg. Chem. 305, 218 (1990); S. I. Filippov, V. I. Antonenko, Nauchn. Dokl. Vysshei Shkoly Met. No. 3, 5 - 9 (1959); V. P. Elyukin, Yu. A. Pavlov. S. B. Sheboldaev, Sb. Mosk. Inst. Stali i Splavov No. 49, 23 - 45 (1968), Y. I sobe, P. Son, M. Miyake, Journal of less common metals 147, 261 - 268 (1989). Ein Überblick findet sich im Gmelin Ergänzungsband A1 zu Molybdän von 1977, Seiten 85 - 87]. Standzeiten von > 3 Jahren bei nur unwesentlich absinkender Zehrung werden so möglich. Das Carbid entsteht durch Reduktion einer Metallverbindung mit dem Kohlenstoff eines stückigen Kohlenstoff-Trägermaterials an dessen Oberfläche und in oberflächennahen Zonen.

Die Erfindung wird nachstehend anhand von Beispielen näher erläutert:

### Beispiele 1 bis 3 (Aktivität von Katalysatorproben)

Zwecks Beurteilung der Aktivität der Katalysatorproben wurden in einem Modellreaktor 100 g Natriumamalgam mit 0,3 Gew.-% Natrium vorgelegt und 10 g Katalysatorkörner in jeweils gleicher Körnung (5 - 10 mm) zugefügt. Darüber wurden 100 ml Alkohol geschichtet. Sowohl das Amalgam als auch der Alkohol wurden gleichzeitig mit demselben Rührer gerührt. Als Maß für die Aktivität diente die zeitliche Zunahme der Leitfähigkeit der sich bildenden alkoholischen Alkoholatlösungen bei 60 °C.
Die so gemessene Aktivität hat sich in der Praxis als proportional zur Raum-Zeit-Ausbeute technischer Reaktoren erwiesen.

In diesem Modellreaktor ergaben sich für verschiedene Katalysatoren folgende Zeitspannen bis zur vollständigen Zersetzung des Amalgams.

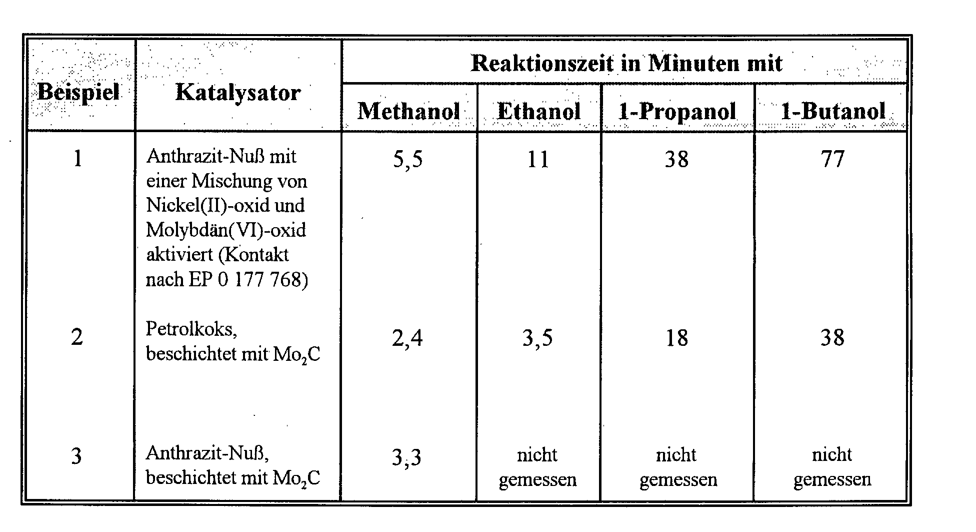

Die beste Zersetzungsleistung mit auf Trägern aufgebrachten Katalysatoren ergibt sich mit dem Katalysator des Beispiels 2. Der sehr viel preiswertere Katalysator des Beispiels 3 ist nur unwesentlich schlechter und erlaubt ebenfalls noch eine praktisch 100%ige Zersetzung. Ethanolat läßt sich erfindungsgemäß mit der Raum-Zeit-Ausbeute herstellen, die vorher nur für Methanolat gegeben war.

## Patentansprüche

1. Verfahren zur katalytischen Herstellung von Alkalialkoholaten aus Alkaliamalgamen und Alkoholen,
dadurch gekennzeichnet,
daß die Umsetzung in Gegenwart von Katalysatoren aus Carbiden und Nitriden der Metalle Chrom und Molybdän, Wolframnitrid sowie Titancarbid durchgeführt wird.

2. Verfahren nach Anspruch 1,
dadurch gekennzeichnet,
daß das Carbid durch Reduktion einer Metallverbindung mit dem Kohlenstoff stückigen Kohlenstoff-Trägermaterial an dessen Oberfläche und in oberflächennahen Zonen entsteht.

3. Verfahren nach einem der vorherigen Ansprüche,
dadurch gekennzeichnet,
daß als Metall Molybdän verwendet wird.

4. Verfahren nach Anspruch 1,
dadurch gekennzeichnet,
daß als Alkoholkomponente aliphatische Alkohole mit 1 bis 4 Kohlenstoffatomen umgesetzt werden.

## Claims

1. A process for the catalytic preparation of alkali metal alkoxides from alkali metal amalgams and alcohols, characterised in that the reaction is carried out in the presence of catalysts selected from carbides and nitrides of the metals chromium and molybdenum, tungsten nitride and titanium carbide.

2. A process according to claim 1, characterized in that the carbide is formed by reduction of a metal compound by the carbon of particulate carbon support material on the surface of the latter and in zones close to the surface.

3. A process according to any one of the preceding claims, characterized in that molybdenum is used as metal.

4. A process according to claim 1, characterized in that aliphatic alcohols having from 1 to 4 carbon atoms are used as alcohol components.

## Revendications

1. Procédé de préparation catalytique d'alcoolates de métal alcalin à partir d'amalgames de métal alcalin et d'alcools,
caractérisé en ce que
la réaction s'effectue en présence de catalyseurs à base de carbures et de nitrures des métaux chrome et molybdène, de nitrure de tungstène ainsi que de carbure de titane.

2. Procédé selon la revendication 1,
caractérisé en ce que
le carbure se forme par réduction d'un composé métallique avec le carbone d'un matériau de support carboné réduit en morceaux, à sa surface et dans les zones proches de la surface.

3. Procédé selon l'une quelconque des revendications précédentes,
caractérisé en ce qu'
on utilise comme métal le molybdène.

4. Procédé selon la revendication 1,
caractérisé en ce qu'
on met à réagir comme composant alcool, des alcools aliphatiques ayant de 1 à 4 atomes de carbone.
